# EUROPEAN PATENT SPECIFICATION

(11) **EP 4 000 523 B1**
(45) Date of publication and mention of the grant of the patent: **06.05.2026**
(21) Application number: 20206849.0
(22) Date of filing: 11.11.2020
(51) Int. Cl.: A61B 5/291, A61B 5/00, A61B 5/372

(54) **METHOD OF FORMING MODIFYING DATA RELATED TO DATA SEQUENCE OF DATA FRAME INCLUDING ELECTROENCEPHALOGRAM DATA, PROCESSING METHOD OF ELECTROENCEPHALOGRAM DATA AND ELECTROENCEPHALOGRAM APPARATUS**
VERFAHREN ZUR BILDUNG VON MODIFIZIERENDEN DATEN, DIE SICH AUF DIE DATENFOLGE EINES DATENRAHMENS BEZIEHEN, DER ELEKTROENZEPHALOGRAMMDATEN ENTHÄLT, VERFAHREN ZUR VERARBEITUNG VON ELEKTROENZEPHALOGRAMMDATEN UND ELEKTROENZEPHALOGRAMMGERÄT
MÉTHODE DE FORMATION MODIFIANT LES DONNÉES LIÉES À LA SÉQUENCE DE DONNÉES D'UNE TRAME DE DONNÉES COMPRENANT DES DONNÉES D'ÉLECTROENCÉPHALOGRAMME, MÉTHODE DE TRAITEMENT DES DONNÉES D'ÉLECTROENCÉPHALOGRAMME ET APPAREIL D'ÉLECTROENCÉPHALOGRAMME

(43) Date of publication of application: 25.05.2022
(73) Proprietor: Cerenion Oy, 90590 Oulu (FI)
(72) Inventor: Väyrynen, Eero, 90590 Oulu (FI); Ala-Kurikka, Jussi, 90590 Oulu (FI); Salminen, Miikka, 90590 Oulu (FI); Tobón, Marcela, 90590 Oulu (FI); Niemelä, Erika, 90590 Oulu (FI)
(74) Representative: Kolster Oy Ab

(56) References cited:
- US-A- 6 042 548
- US-A1- 2004 010 203
- US-A1- 2013 023 783
- US-B1- 7 672 717
- : JOSE ANTONIO URIG�EN AND BEGO�A GARCIA-ZAPIRAIN: "EEG artifact removal state-of-the-art andguidelines", vol. 12, no. XP0202895997, 1 January 2015 (2015-01-01), Retrieved from the Internet <URL:https://iopscience.iop.org/article/10.1088/1741-2560/12/3/031001> [retrieved on 20250610]
- BAHADOR, NOOSHIN AND JOKELAINEN, JARNO AND MUSTOLA, SEPPO AND KORTELAINEN, JUKKA: "Reconstruction of missing channel in electroencephalogram using spatiotemporal correlation-based averaging", vol. 18, no. 5, 5 October 2021 (2021-10-05), XP093342067, Retrieved from the Internet <URL:https://doi.org/10.1088/1741-2552/ac23e2> [retrieved on 20211005], DOI: 10.1088/1741-2552/ac23e2

## Description

### Field

The invention relates to a method of forming modifying data related to a data sequence of a data frame including electroencephalogram data, and an electroencephalogram apparatus.

### Background

Electroencephalography (EEG) signals can be analysed by performing a qEEG (quantitative EEG) analysis using algorithms of computer programs. However, such a computer based analysis is sensitive to situations where some raw EEG data is missing because of a data sequence lost due to communication errors, for example. Alternatively or additionally, the raw EEG data may be contaminated by artefacts. Such undesired phenomena may render the EEG analysis incalculable or result in a large analysis error. The resending of lost or contaminated data is not always practical or possible. The reason may be that the analysis needs to be done in real-time, for example. Sometimes the data frame requires an additional data sequence in order to be analysed.

Patent document US7672717 presents a method and a system for the denoising of large-amplitude artifacts in electrograms using time-frequency transforms.

Patent document US6042548 presents a virtual neurological monitor and method.

Urigüen, J.A., Garcia-Zapirain, B., EEG artifact removal-state-of the-art and guidelines, Journal of Neural Engineering, Vol. 12, No. 3, 2015, Article 031001. DOI: 10.1088/1741-2560/12/3/031001 presents a review on the artifact removal algorithms used to remove the main sources of interference encountered in the electroencephalogram (EEG), specifically ocular, muscular and cardiac artifacts.

The prior art has attempted to solve the problem by replacing a lost or contaminated data sequence of an EEG data frame by a copy of a properly received data sequence of an EEG data frame which is contamination free or by adding a proper EEG data frame to the data frame. However, such a substitution is also known to result in a large analysis error. Hence, the present signal processing is inadequate and an improvement would be welcome.

Bahador, Nooshin and Jokelainen, Jarno and Mustola, Seppo and Kortelainen, Jukka: "Reconstruction of missing channel in electroencephalogram using spatiotemporal correlation-based averaging",vol. 18, no. 5 5 October 2021 (2021-10-05),DOI: 10.1088/1741-2552/ac23e2. Internet:URL:https:// doi.org/10.1088/1741-2552/ac23e2 teaches correlation-based averaging of neighbouring channels, producing one deterministic reconstruction of missing or corrupted EEG data according to prior art.

### Brief description

The present invention seeks to provide an improvement in the formation of a data sequence and processing method of the data frame(s).

The invention is defined by the independent claims. Embodiments are defined in the dependent claims.

### List of drawings

Example embodiments of the present invention are described below, by way of example only, with reference to the accompanying drawings, in which
Figure 1 illustrates an example of a data frame of a signal with a unusable data sequence, where data may be missing or corrupted;
Figure 2 illustrates an example of a data frame with a copy of an available data sequence of the data frame is pasted in a location of a corrupted or missing data sequence;
Figure 3 illustrates an example of a data frame with a shuffled copy of an available data sequence of the data frame is pasted in a location of a corrupted or missing data sequence;
Figure 4 illustrates an example of a data frame with a copy of another available data sequence of the data sequence is pasted in a location of a corrupted or missing data sequence;
Figure 5A illustrates an example of a neutral data sequence of a good fit pasted in a location of a corrupted or missing data sequence;
Figure 5B illustrates an example of a data frame pasted in front of the data sequence of the data frame;
Figure 6A illustrates an example how process is performed;
Figure 6B illustrates an example of selection of a surrogate algorithm for forming a neutral data sequence;
Figure 6C illustrates an example of selection of a neutral data sequence from a plurality of candidates;
Figure 7 illustrates an example of electroencephalographic measurement with a data processing unit comprising at least one processor and at least one memory;
Figure 8 illustrates of an example of a flow chart of a method to form modifying data for a data frame including electroencephalogram data;
Figure 9 illustrates an example of a flow chart of a method utilizing at least one first parameter, which defines the surrogate algorithm of candidates of neutral data sequences;
Figure 10 illustrates an example of of a flow chart of a method of a processing method of electroencephalogram data.

### Description of embodiments

The following embodiments are only examples. Although the specification may refer to "an" embodiment in several locations, this does not necessarily mean that each such a reference is to the same embodiment(s), or that the feature only applies to a single embodiment. Single features of different embodiments may also be combined to provide other embodiments. Furthermore, words "comprising" and "including" should be understood as not limiting the described embodiments to consist of only those features that have been mentioned and such embodiments may also contain features/structures that have not been specifically mentioned. All combinations of the embodiments are considered possible if their combination does not lead to structural, operational or logical contradiction.

It should be noted that while Figures illustrate various embodiments, they are simplified diagrams that only show some structures and/or functional entities. The connections shown in the Figures may refer to logical or physical connections. It should be appreciated that details of some functions, structures, and the signalling used for measurement and/or controlling are irrelevant to the actual invention. Therefore, they need not be discussed in more detail here.

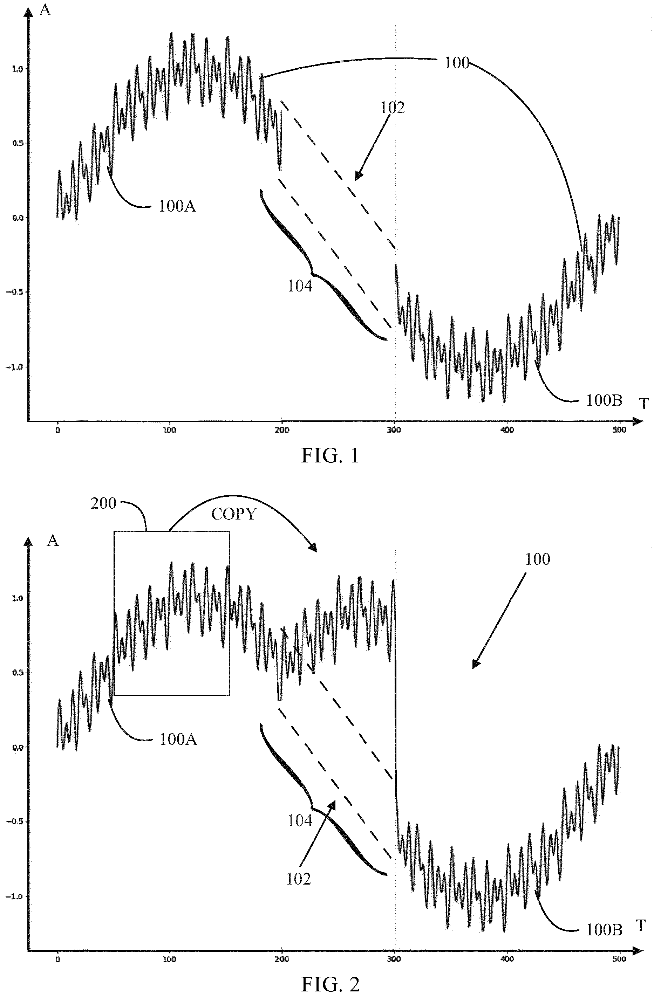

Figure 1 illustrates an example of a data frame 100 of a signal that has a data sequence 102 missing, from an analysis point of view. The data sequence 102 may instead of being missing be corrupted. The corruption may be caused by contamination by other signals or noise, or by alterations that are results of data transfer, recording and processing and that prevent a proper use of the data thereafter. The data sequence 102 may in some cases have one or more undesired artefacts causing a loss of a required data. The data sequence 102 may be unusable for data processing. The data sequence 102 may be unavailable because it may have been partly or fully unsent or undetected. Hence, the data sequence 102 may be called a corrupted or missing data sequence.

The vertical y-axis denotes amplitude or power A and the horizontal x-axis denotes time T. Both axes are in arbitrary scales. In this example, the data frame 100 comprises the signal as a time series.

The dashed lines at the gap 104 show possible outlines or envelope of a signal portion that may be suitable to fill the gap 104 between the proper signal portions 100A and 100B of the data frame 100. The dashed lines may serve as outlines of a reference.

Figure 2 illustrates an example where the corrupted or missing data sequence 102 has been replaced by a copy of an available data sequence 200 of the data frame 100, or the copy of the available data sequence 200 has been inserted in a location of the corrupted or missing data sequence 102. When it is missing it may be missing fully or partly. If it is missing partly, it can also be understood to be corrupted. The available data sequence is non-lost and its corruption is below a predetermined threshold or it is non-corrupted.

Like in Figure 1, the vertical y-axis denotes amplitude or power A and the horizontal x-axis denotes time T. Both axes are in arbitrary scales.

As can be seen in this example, it does not fit within the dashed lines relating to the reference, which can be used as a visualization example that a mean, a standard deviation and/or some other statistical parameter of the data frame 100 may be disturbed when a copy of a signal portion 200 is added to a data frame 100 or a data sequence 102 is replaced by a copy of another data sequence of the data frame 100. Such a disturbance may cause error to a result that is measured from the data frame 100.

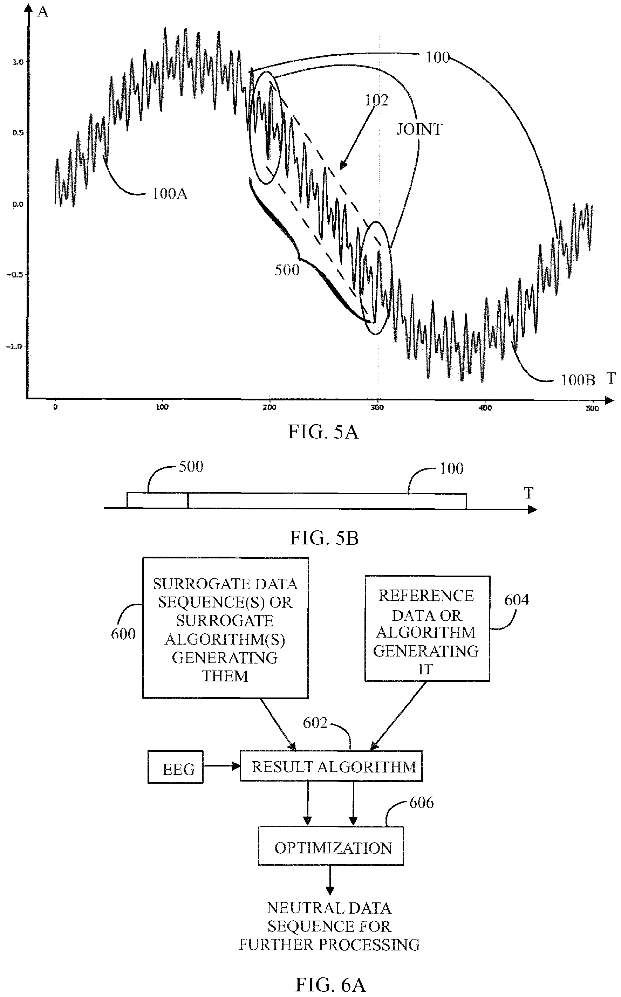

However, this method of pasting a copy of an available data sequence 200 in a location of a corrupted or missing data sequence 102 (or in front of the data frame), *per se,* works and the copy of the available data sequence 200 is used like or as one of the neutral data sequences 500 (see Figure 5A) if the copy is properly selected i.e. the selection is performed under proper selection rules or using a proper computation algorithm. Then the rules/algorithm is linked to an analysing algorithm of the data frame that generates the result. Namely the optimization under the proper selection rules or under the proper computation attempts to find a local or global minimum for finding a suitable neutral data sequence 500. According to the invention, the optimization tries to balance between quality and speed, where the quality referring to close to an absolute minimum of the found neutral data sequence 500 is.

In general, modifying data related to a data sequence 102 that is missing or corrupted in the data frame 100 including electroencephalogram data is formed in a following manner. The modifying data is a neutral data sequence 500 or a surrogate algorithm 652 that generates the neutral data sequence 500 by forming at least one surrogate data sequence 662.

The modifying data is formed by selecting one sequence from at least one surrogate data sequence 662 and the selected one becomes a neutral data sequence 500. The neutral data sequence 500 is used to replace a missing or corrupted data sequence 102 of the electroencephalogram data. Alternatively the modifying data is formed by selecting a surrogate algorithm 652 from at least one surrogate algorithms 652, each of which is used for generating the surrogate data sequences 662. The selection is based on an optimization comparison 606 between a first data and a second data in order to limit disturbance caused by application of the modifying data to the data frame 100. Disturbance may be caused if the neutral data sequence 500 is inserted to the data frame 100, or if the neutral data sequence 500 is formed by the surrogate algorithm 652 and inserted the neutral data sequence 500 to the data frame 100.

The first data comprises reference data or a reference algorithm for generating the reference data.

The second data comprises at least one result formed by applying a result algorithm, which provides characterizing information on the data frame 100 including the electroencephalogram data, to the electroencephalogram data with the at least one surrogate data sequence 662, which can replace the missing or corrupted data sequence of the electroencephalogram data. Alternatively, the second data comprises the result algorithm that is for generating the at least one result from the electroencephalogram data with the at least one surrogate data sequence 662. Still alternatively, the second data comprises the result algorithm with the electroencephalogram data and the at least one surrogate algorithm 652 that is for providing the at least one surrogate data sequence 662. The result algorithm has the electroencephalogram data and the at least one surrogate algorithm 652 as if its arguments. The optimization comparison will result in the neutral data sequence 500 or the surrogate algorithm 652 that provides the neutral data sequence 500.

In Figure 2, the copy of the available data sequence 200 may be one of the at least one surrogate data sequence 662.

In an embodiment, the available data sequence 200 or any surrogate data sequence 662 may comprise the same number of bits as that of the corrupted or missing data sequence 102. The available data sequence 200 may be before or behind the corrupted or missing data sequence 102.

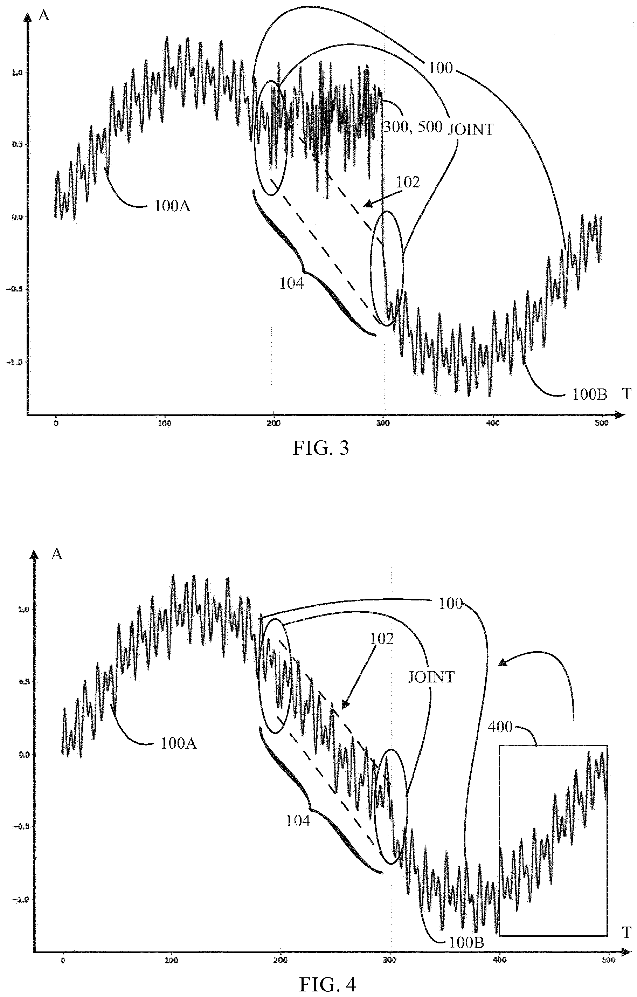

Figure 3 illustrates an example where the corrupted or missing data sequence 102 is replaced by a shuffled copy of an available data sequence 300 or the shuffled copy of the data sequence 300 may be inserted in a location of the corrupted or missing data sequence 102 that may be missing fully or partly. In this example a half of the copy of the available data sequence 300 is the same as that in Figure 2 but another half of the copy of the available data sequence 300 is taken from behind the corrupted or missing data sequence 102.

The vertical y-axis denotes amplitude or power A and the horizontal x-axis denotes time T. Both axes are in arbitrary scales.

The shuffled copy of the available data sequence 300 of Figure 3 fits slightly better in the location of corrupted or missing the data sequence 102, whose outlines are shown with the dashed lines of a reference, than in the example of Figure 2. This replacement, *per se,* may also work and the copy of the available and shuffled data sequence 300 may be used like or as one of the neutral data sequence 500 (see Figure 500) if a result of the analysing algorithm becomes acceptable. However, a copy of an available and shuffled data sequence from any location of the data frame 100 is not necessarily suitable for its purpose. The optimization of the data sequence to be pasted should depend on the rules/algorithm that can be linked to an analysing algorithm of the data frame that generates the result for a further use/processing.

In general, the shuffling refers to a randomization of bits of a time series. That is, an order of bit in the time series is changed such that they become random or pseudorandom using a predetermined pseudo-random indexing sequence (in this way the at least one surrogate data sequence 662 is also deterministically reproducible). As an example, the shuffling may be performed using DFFT (Digital Fast Fourier Transform). The copy of the available data sequence may be DFFT transformed first. Then random phases may be substituted for complex components. Finally, the data sequence with (pseudo)random phases may be inverse digital fast Fourier transformed (IDFFT).

Another possibility is to use an iteratively refined surrogate method, where DFFT transformed at least one surrogate data sequence 662 having iteratively adjusted amplitudes are used for optimization. In this manner, the linear statistical and spectral properties (such as autocorrelation, mean, power spectrum, probability distribution, variance or the like) in the at least one surrogate data sequences 662 can be kept unchanged.

The at least one surrogate data sequence 662 may be formed by interpolation (for short gaps, for example) or with so called surrogate data generation methods (for larger gaps, for example) using as a base the available data of the data frame 100 and/or some other specified suitable data. For example, a suitable length of previous data frame may be shuffled and then injected to replace the unusable data sequence 102. The other specified suitable data may include data that has been collected in conditions that are sufficiently similar to the conditions of the measured encephalographic signal.

Using the shuffling (see Figure 3), not only the mean and standard deviations of the produced neutral data sequence 500 are good estimates for sequence of nearby real data, but also correlations between data channels of the encephalogram measurement are removed when the shuffling is done independently for each channel. The replacement data may also be selected from a nearby valid data following the gap if the gap is too long for a simple interpolation, the causality requirements are not strict and/or otherwise the signal quality after injection is more desirable (e.g. trend continuity is desired, or sharp edges are not desired at the gap boundaries)

Naturally, the shuffled copy of the data sequence 300 may be one of the at least one surrogate data sequence 662 for the optimization.

Figure 4 illustrates an example where the data sequence 102 is replaced by a copy of an available data sequence 400 or the copy of the available data sequence 400 is inserted in a location of the corrupted or missing data sequence 102. In this example, the copy of the available data sequence 400, in this example not shuffled, has been taken from another location than in the examples of Figures 2 and 3. However, the copy may be modified to a certain extent without shuffling.

The vertical y-axis denotes amplitude or power A and the horizontal x-axis denotes time T. Both axes are in arbitrary scales.

The copy of the available data sequence 400 of Figure 4 fits better in the location of the corrupted or missing data sequence 102, whose expected outlines are shown with the dashed lines, than in the examples of Figures 2 and 3. This replacement, *per se,* may also work and the copy of the available data sequence 400 may be used like or as a neutral data sequence 500 (see Figure 500) if a result of the analysing algorithm becomes acceptable. The optimization of the data sequence to be pasted should depend on the rules/algorithm that can be linked to an analysing algorithm of the data frame that generates the result for a further use/processing.

Naturally, the copy of the available data sequence 400 may be one of the at least one surrogate data sequence 662.

Figure 5A illustrates an example where the corrupted or missing data sequence 102 is replaced by a neutral data sequence 500 or the neutral data sequence 500 has been inserted in a location of the corrupted or missing data sequence 102. This example illustrates an example where the neutral data sequence 500 is based on the optimization, the optimization having stricter requirements for the replacement or the insertion in this example than in Figures 2 to 4.

The vertical y-axis denotes amplitude or power A and the horizontal x-axis denotes time T. Both axes are in arbitrary scales.

The neutral data sequence 500 fits well in the location of the corrupted or missing data sequence 102 whose expected outlines are shown with the dashed lines which may be linked to the reference. Figure 5 also serves visually as an example that a desired parameter of the data frame 100 may be measured based on the optimization such that the pasted neutral data sequence 500 disturbs the measurement minimally because the neutral data sequence 500 is a good match with the expected original data sequence of the data frame 100.

In an embodiment, the derivative at one side and a derivative of another side of a joint between the original data sequence 100A, 100B and the neutral data sequence 500 may be made at least about the same with the optimization. That is, the curve of the data frame 100 is not only continuous but also its derivative may be continuous at the joint. Then little or no mismatch can be observed at the joint, and an analysis of the data frame 100 may be more reliable.

Figure 5B illustrates an example where the neutral data sequence 500 may be inserted in front of the data frame 100. Correspondingly, the at least one surrogate data sequence 662 or after the optimization comparison the neutral data sequence 500 may be pasted in front of the data sequence 100. In this example, the unusable data sequence 102 can be understood to missing because the result algorithm may require it in certain cases to be applicable. The horizontal x-axis denotes time T and it is in an arbitrary scale. In an embodiment, the neutral data sequence 500 may be inserted behind the data frame 100 (not shown in Figures).

In an embodiment, an average slope of the original data sequence 100A, 100B and the neutral data sequence 500 at the joint may be made at least about the same with the optimization. Then little or no mismatch can be observed at the joint, and an analysis of the data frame 100 including the electroencephalogram may be more reliable. The analysis of the data frame may be based on a result algorithm that provides characterizing information on the data frame 100. The characterizing information may include at least one measured parameter. The characterizing information may refer to a mean, standard deviation, correlation, coupling, signal-to-noise ratio, autocorrelation (of a recurring signal), frequency, frequency distribution, spectral information, amplitude, power, phase, one or more statistical moments etc., for example.

The statistical moments are the following: the zeroth moment is an overall probability, the first moment is the expected value, the second moment is the variance, the third moment is the skewness, and the fourth moment is the kurtosis.

In general, note that the neutral data sequence 500 may be added to a beginning of the data frame 100, somewhere between the beginning of the data frame 100 (like in examples of Figures 2 and 3), and/or an end of the data frame 100 or at the end of the data frame 100. The unusable data sequence 102 may be unavailable because it may have been unsent or undetected. The surrogate data sequences 662 may be added at the beginning of a signal in front of the data frame 100.

According to the invention, the at least one surrogate data sequence 662 is formed from the one or more original data sequences 100A, 100B by phase randomization. The surrogate data sequences 662 may have the same power spectrum as the one or more original data sequences 100A, 100B, which means that their linear correlations do not differ from each other within a tolerance. In this manner, the power spectrum amplitudes of the data frame 100 is preserved while randomizing phase of the signal of the data frame 100.

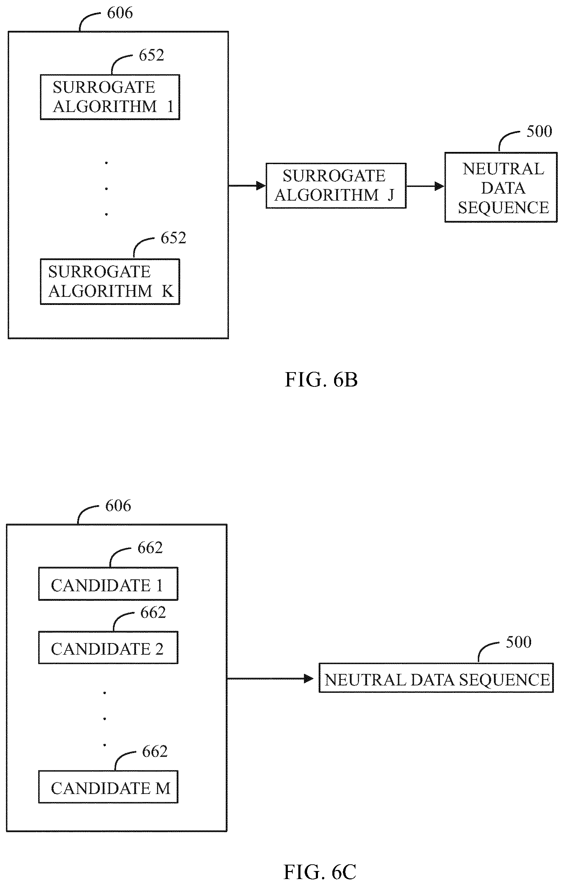

The following explains based on an example shown in Figure 6A how a proper neutral data sequence 500 for a data frame 100 including electroencephalogram data is formed or found. As shown in block 600, plurality of surrogate data sequences 662 (see Figure 6C) or one or more surrogate algorithms 652 (see Figure 6B) that generate a plurality of the surrogate data sequences 662 is available.

Then the result algorithm of block 602 that provides characterizing information on the data frame 100 including the electroencephalogram data should also be available. The result algorithm may provide statistical information on the data frame 100. The information includes frequency, frequency distribution, spectral information, amplitude, power, phase, one or more statistical moments of at least one of these or the like, for example. Additionally or alternatively, the result algorithm provides information on burst suppression and/or quality of the electroencephalogram data, for example. The quality may refer to a feature that the electroencephalogram data may fit in a predetermined model or the feature may be an envelope of the electroencephalogram data.

Results by applying the result algorithm to a combination of each of the surrogate data sequence 662 and the data frame 100 is then formed or enabled. Alternatively, a combination of each of the surrogate algorithm 652 and the data frame 100 is then formed or enabled.

A corresponding reference result is formed by applying the result algorithm to reference data, which corresponds to the electroencephalogram data of the data frame 100. Instead of reference data, *per se,* one or more reference algorithms that generate the reference data is available for the result algorithm. Both of these possibilities are shown in block 604. That is, the reference data may have real electroencephalogram data that is not corrupted nor has missing portions from the analysis point of view. The correspondence means that electroencephalogram data of the reference may have been measured from the same location(s) of the brain in the same conditions as the electroencephalogram data of the data frame 100. Alternatively, the reference is formed as a simulation providing a time series similar to electroencephalogram data measured from the same location(s) of the brain in the same conditions as the electroencephalogram data of the data frame 100 to be analyzed. In an embodiment, a plurality of electroencephalogram data may be averaged for the reference. The reference should be such that it is similar to a typical electroencephalogram data and/or it provides similar couplings between the channels as a typical electroencephalogram signal. A typical electroencephalogram signal, according to the invention is that measured from a healthy person or an average of electroencephalogram signals measured from a healthy person. Alternatively or additionally, the reference includes an electroencephalogram signal that is formed based on a model that generates a typical electroencephalogram signal. Finally, a suitable neutral data sequence 500 is formed or selected by optimization comparison 606 based on the result algorithm, the data frame 100 from the EEG, the at least one surrogate algorithm 652 and/or the at least one surrogate data sequence 662, and the reference algorithm and/or the reference data in block 606. The optimization comparison optimizing an error between the results formed by applying the result algorithm to the data frame with the at least one surrogate data sequence 662 and to the reference data in block 606. The surrogate algorithm as well as the surrogate data sequences 662 may be deterministic, pseudorandom or random and particularly deterministic, pseudorandom or random data sequences, which makes it possible to select them based on a suitable criterion. For example, simulations made before an actual search for the neutral data sequence 500 can be useful to reveal which surrogate algorithm should be used in a certain situation. That is like calibration of the method and/or apparatus for its actual use.

Finally, a suitable neutral data sequence 500 is formed or selected by optimizing error between the results formed by applying the result algorithm to the data frame with the at least one surrogate data sequence 662 and to the reference data in block 606.

Figure 6B illustrates an example of selection of a surrogate algorithm 652 for forming a neutral data sequence. The optimization comparison may select one of the surrogate algorithm 652 from a group surrogate algorithms 652 each of which is configured to form a surrogate data sequence 662. The selected surrogate algorithm, that is J^{th} algorithm in an order of the surrogate algorithms, can then form the neutral data sequence 500. A number of the surrogate algorithms 652 may be K, where K is a whole number equal to or larger than one, for example, and 1 ≤ J ≤ K. In an embodiment, the number K is two or larger.

Figure 6C illustrates an example of selection of a neutral data sequence 500 from a plurality of candidates of the at least one surrogate data sequence 662 on based on the optimization comparison 606. In an embodiment, the number of the at least one surrogate data sequence 662 is M, which may be a whole number equal or larger than 2. In an embodiment, the number M of the at least one surrogate data sequence 662 may be at least one. Namely, even only one surrogate data sequence 662 can be tested if it passes the optimization comparison.

In other words, the neutral data sequence 500 for a corrupted or missing data sequence 100 of electroencephalogram data is formed by optimizing error between results, which are formed by applying a result algorithm to a combination of the electroencephalogram data and at least one surrogate data sequence 662, and a corresponding reference result. A single combination may include the electroencephalogram data and one of the at least one surrogate data sequence 662 in the location of the corrupted or missing data sequence 102. Another single combination may include the electroencephalogram data and another one of the at least one surrogate data sequence 662 in the location of the corrupted or missing data sequence 102. The result algorithm may be applied to whole available electroencephalogram data of the data frame 100 or to a portion of the electroencephalogram data of the data frame 100 in the optimization process.

The neutral data sequence 500 and the data frame 100 including the electroencephalogram data may be utilized in a further processing.

The surrogate data sequences 662 are approximations that try predict the behaviour of the signal within corrupted or missing data sequence 102 of the data frame 100 as closely as possible. One of the surrogate data sequences 662 can then be accepted and set as the neutral data sequence 500 in the optimization. If the optimization is performed using artificial intelligence, it will generate or predict (e.g. hallucinate) the neutral data sequence 500 for the data frame 100 on the basis of the evaluation rules of the optimization.

In this manner, i.e. when adding a randomly, pseudorandomly or deterministically formed neutral data segment 500, the computation of the analysis with residual analysis error is wanted to be as low as possible compared to the analysis result when full data would be available. The inserted neutral data sequence 500 may be formed in such a way that features and properties of the neutral data sequence 500 affect as little as possible the calculations of the target analysis (or analysis in a chain). Typically, the desired properties of the neutral data sequence 500 are close to the amplitudes and spectral distributions of nearby valid data sequences 100A, 100B.

For the result algorithm calculating the standard deviation of the data frame 100 in an embodiment, the neutral data sequence 500 should have the mean and standard deviation matching the means and standard deviations of the nearby original data sequences 100A, 100B. At least partly, this comes advantageously from the use of the reference.

In an embodiment, when analyzing the correlation of two EEG data channels, the neutral data sequence 500 of each of the channels may be set to have no or negligible inter-channel correlations. At least partly, this comes advantageously from the use of the reference.

In an embodiment, when the spectral properties are important for an analysis, for example, a power spectrum of the neutral data sequence 500 may be set to as close as possible to that of the nearby original data sequences 100A, 100B.

In an embodiment, the data frame 100 including the electroencephalogram data and the neutral data sequence 500 may be combined.

In an embodiment, the optimization of the error is performed by substituting a corrupted or missing data sequence 102 of the electroencephalogram data 100 with the at least one surrogate data sequence 662 for forming the results by applying the result algorithm to the electroencephalogram data with the at least one surrogate data sequence 662. Then the missing or corrupted data sequence 102 of the electroencephalogram data may be replaced by a neutral data sequence fulfilling the optimization.

In an embodiment, the optimization of the error is performed by substituting a corrupted or missing data sequence 102 of the electroencephalogram data 100 with only a single surrogate data sequence 662 for forming the result by applying the result algorithm to the electroencephalogram data with the single surrogate data sequence 662. Then, if the single surrogate data sequence 662 is acceptable, the corrupted or missing data sequence 102 may be replaced by the surrogate data sequence 662 fulfilling the optimization, the surrogate data sequence 662 thus becoming the neutral data sequence 500.

In an embodiment, electroencephalogram data may be received from a plurality of electroencephalogram channels. The plurality of electroencephalogram channels may be processed as a vector such that a data frame 100 of a single channel of the plurality of the electroencephalogram channels is processed as a single element of the vector. Then the optimization comparison 606 may be performed to at least one element i.e. channel of the vector. That is, at least one of the data frames 100 of the at least one electroencephalogram channel is processed individually in order to have a neutral data sequence of the optimization in a location of a corrupted or missing data frame 100.

In an embodiment, the at least one surrogate data sequence 662 are formed using a surrogate algorithm 652, which utilizes the electroencephalogram data that is uncorrupted and available. The surrogate algorithm 652 may be deterministically dependent on the result algorithm or it may be independent of it. That is, the surrogate algorithm 652 may vary with the result algorithm. In an embodiment, a selection algorithm that selects available data sequences from the data frame 100. The selection may be deterministic, pseudorandom or random. If a corrupted or missing data sequence 102 is in a location where amplitude is becoming higher (becoming lower), the selection algorithm may select an available data sequence or a combination of available data sequences that has/have a becoming higher (becoming lower) amplitude, for example. In an embodiment, the surrogate algorithm 652 may be a simulation algorithm that generates surrogate data sequences 662.

In an embodiment, at least one first parameter, which defines the surrogate algorithm 652, may be formed based on the result algorithm. The at least one parameter may define a frequency or frequency band of the surrogate data sequences 662 that the surrogate algorithm 652 may generate. The surrogate data sequences 662 may then be formed using the surrogate algorithm 652 determined by the at least one first parameter for the optimization, the surrogate algorithm 652 being random, pseudorandom or deterministic. The data frame 100 including the electroencephalogram data and a neutral data sequence 500 formed by the optimization may then be combined.

In an embodiment, the at least one first parameter of the surrogate algorithm 652 may be determined based on at least one frame parameter of the data frame 100, where the at least one frame parameter may define or relate to a property of the data frame. The at least one frame parameter may be a length, frequency, frequency band, frequency distribution, amplitude, phase, power, power spectrum, and/or curvature of the signal of the data frame 100.

In an embodiment, the at least one first parameter of the surrogate algorithm 652 may be determined based on at least one second parameter of the result algorithm, and the at least one second parameter may define the result algorithm.

In an embodiment, the at least one second parameter of the result algorithm may comprise at least one of the following: a correlation between channels, a frequency distribution, a frequency band, an amplitude, a phase and a length of the corrupted or missing data sequence 102. The frequency distribution, the frequency band, the amplitude and the phase may relate to the non-missing and/or non-corrupted section(s) of the data frame 100.

In an embodiment, a neutral data sequence is selected from a set of surrogate data sequences 662, which are predetermined.

In an embodiment, at least one reference result may be formed for the optimization comparison by applying the result algorithm to at least one model electroencephalogram data, a single reference result corresponding to a single model electroencephalogram data of the at least one model electroencephalogram data, and/or by utilizing at least one reference algorithm that provides at least one reference result similar to those formed by applying the result algorithm to at least one model electroencephalogram data.

In an embodiment, a plurality of the neutral data sequences 500 may be formed for a plurality of the data frames 100. Then a single neutral data sequence of the surrogate data sequences 662 that fulfils the optimization comparison criterion or optimizes the error is selected for any one of the data frames 100 based on the optimization comparison. Finally, the single neutral data sequence of one of the data frames may be the same as or different from that of another of the data frames.

In an embodiment, the result algorithm may form a first moment, a second moment, a third moment, a fourth moment and/or a median of statistical parameters when applied to the data frame 100 having the neutral data sequence 500.

In an embodiment, the surrogate data sequences 662 may be formed by interpolating the electroencephalogram data and/or by applying one or more surrogate data generation methods.

In an embodiment, the optimization may be performed by minimizing error of at least one of the first moment, the second moment, the third moment, the fourth moment and/or the median of statistical parameters of a set of combinations of the electroencephalogram data and the at least one surrogate data sequence 662.

Consider now some aspects of the processing solution of forming a neutral data sequence 500 for a data frame 100 including electroencephalogram data. When the real-time requirements are essential the method for forming the neutral data sequence 500, the at least one surrogate data sequence 662 may be chosen or generated such that the computational efficiency of the method is also suitable. The neutral data sequence 500 for the data frame 100 including the electroencephalogram data may be formed while a measurement of the electroencephalogram data or transfer thereof is on-going.

The real-time requirements may require a compromise with any other performance specifications for the surrogate solution. The presented shuffle surrogate method is an example of a computationally efficient solution. However, the spectral density distribution of the shuffled neutral data sequence 500 differs from the original data (i.e. spectrum is flattened by the shuffling and power is typically shifted to higher frequencies). The difference in spectrum is however not necessarily an issue if the bandwidth of the target analysis is low (as it often tends to be in the EEG measurements). If the spectral density is an important property for the analysis then the surrogate method used may need to be selected such that the original spectrum is better estimated using for example a neutral data sequence 500 of the Fourier Transform (FT) surrogate, Amplitude Adjusted Fourier Transform (AAFT), or a neutral data sequence 500 based on an adaptive filter estimate of the previous data power spectrum (Digital Filtered Surrogate, DFS). More advanced methods are also the iterative versions of the AAFT and DFS but these may have higher computational requirements.

The optimization may be tuned using artificial intelligence, neural network and/or machine learning methods, for example, such that the errors of the result algorithm are minimized or some other desired property in the result algorithm is met. The artificial intelligence, neural network and/or machine learning methods may continuously predict or hallucinate a neutral data sequence that can replace the missing or corrupted data, and when a missing or corrupted data is detected, the neutral data sequence will be used to replace the missing or corrupted data sequence. The artificial intelligence, neural network and/or machine learning method may learn the properties of the electroencephalogram data and improve its predictions with the increasing information on the electroencephalogram data.

In an embodiment, an adversarial machine learning approach, which may also utilize an artificial intelligence, neural network and/or machine learning methods, or may be a simpler model or meter, may compete with the artificial intelligence, neural network and/or machine learning, and tries to determine if the neutral data sequence formed by the artificial intelligence, neural network and/or machine learning is authentic i.e. acceptable or not.

If the adversarial machine learning approach or the like determines the formed neutral data sequence authentic or acceptable, the neutral data sequence may replace the missing or corrupted data sequence with the neutral data sequence.

In this way boundaries, where within the analysis results become acceptable (e.g. maximum size of gap 104 allowed to fill, and the amounts and locations suitable data for the forming of the at least one surrogate data sequence 662) may be selected. The boundaries may not only be strongly dependent on the interpolation and generation methods of the at least one surrogate data sequence 662 but also depend on the EEG signal features (e.g. EEG amplifier properties, applied filters, or channel combinations and types).

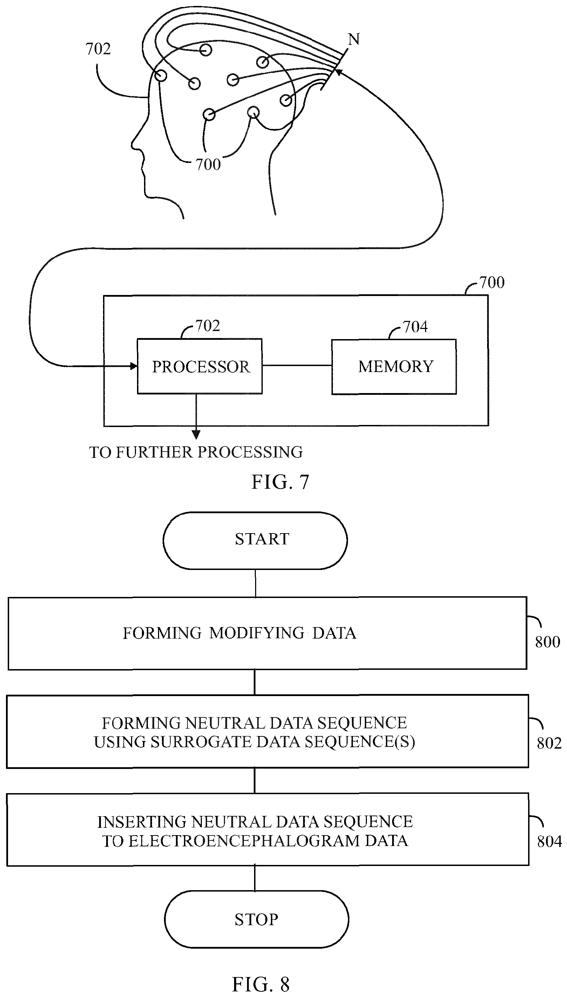

Figure 7 illustrates an example of an electroencephalogram measurement. Electrodes 700 are measuring the electroencephalogram signals from a person's head 702 and the electroencephalogram signals are output in N electroencephalogram channels to a data processing unit 700. The data processing unit 700 may comprise at least one processor 702 and at least one memory 704 which may include a suitable computer program for performing the processing method of forming a neutral data sequence 500 for a data frame 100 including the electroencephalogram data. The data processing unit 700 may comprise an A/D converter that converts the analog forms of the electroencephalogram signals into digital time series.

Figure 8 shows a flow chart of a method of the formation of a neutral data sequence for a data frame 100 including electroencephalogram data according to the present invention. In step 800, the modifying data is formed by selecting one sequence from at least one surrogate data sequence 662 for a neutral data sequence 500 that is for replacing a missing or corrupted data sequence of the electroencephalogram data or selecting a surrogate algorithm, which is for generating the surrogate data sequence 662, by performing an optimization comparison 650 between a first data and a second data in order to limit disturbance caused by application of the modifying data to the data frame 100:
the first data comprising reference data or a reference algorithm for generating the reference data, and the second data comprising at least one result formed by applying a result algorithm, which provides characterizing information on the data frame 100 including the electroencephalogram data, to the electroencephalogram data with the at least one surrogate data sequence 662 replacing the missing or corrupted data sequence of the electroencephalogram data, or the result algorithm that is for generating the at least one result from the electroencephalogram data with the at least one surrogate data sequence 662 or the result algorithm based on the electroencephalogram data and the surrogate neutral data algorithm that is for providing the at least one surrogate data sequence 662.

In step 802 the neutral data sequence 500 is formed by optimizing an error between the first and second data in the optimization comparison, the first data being formed by applying the result algorithm to the electroencephalogram data and the at least one surrogate data sequence 662, and a corresponding reference result, which is formed by applying the result algorithm to the reference data, which corresponds to the electroencephalogram data, for further processing utilizing the neutral data sequence 500 and the data frame 100 including the electroencephalogram data.

In step 804 which is optional, the data frame 100 including the electroencephalogram data and the neutral data sequence 500 are combined.

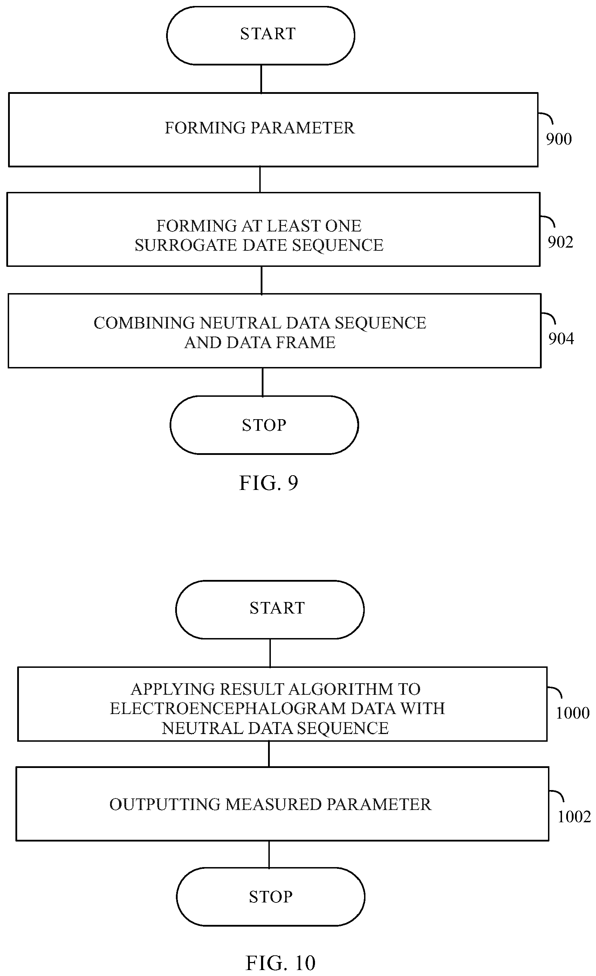

Figure 9 is a flow chart of a method of utilizing at least one first parameter, which defines the surrogate algorithm 652. In step 900, at least one first parameter, which defines the surrogate algorithm 652, is formed, based on the result algorithm. In step 902, the at least one surrogate data sequence 662 are formed using the surrogate algorithm 652 determined by the at least one first parameter for the optimization, the surrogate algorithm 652 being random, pseudorandom or deterministic. In step 904 which may be optional, the data frame 100 and a neutral data sequence 500 formed by the optimization comparison 606 are combined.

Figure 10 shows a flow chart of a processing method of electroencephalogram data. In step 1000, the result algorithm is applied to the electroencephalogram data with the neutral data sequence for providing at least one measured parameter, the neutral data sequence for the electroencephalogram data being formed according to the method of Figure 8. In step 1002, the at least one measured parameter is output.

In general, the methods shown in Figures 8 to 10, according to the invention are implemented as a logic circuit solution or a computer program. The computer program is placed on a computer program distribution means for the distribution thereof. The computer program distribution means is readable by a data processing device, and it encodes the computer program commands, carries out the formation of the neutral data sequence for a data frame 100 including electroencephalogram data and optionally.

The computer program may be distributed using a distribution medium which may be any medium readable by the controller. The medium may be a program storage medium, a memory, a software distribution package, or a compressed software package. In some cases, the distribution may be performed using at least one of the following: a near field communication signal, a short distance signal, and a telecommunications signal.

The invention is defined by the appended cliams.

## Claims

1. A computer-implemented method of forming modifying data related to a data sequence for a data frame (100) including electroencephalogram data, **characterized by**
forming (800) the modifying data by performing a selection of one sequence from at least one surrogate data sequence (662), where the selected one becomes a neutral data sequence (500), the neutral data sequence (500) replacing a missing or corrupted data sequence of the electroencephalogram data, by
forming (802) the neutral data sequence (500) by optimizing an error between a first data and a second data, wherein the first data comprises a reference result formed by applying a result algorithm to reference data, which corresponds to electroencephalogram data that is non-corrupted and without missing portions, and the second data comprises at least one result each formed by applying the result algorithm to the electroencephalogram data with the one sequence from the at least one surrogate data sequence (662) in the location of the corrupted or missing data sequence (102), the result algorithm providing characterizing information on the data frame (100) including the electroencephalogram data, wherein forming the neutral data sequence (500) comprising
performing the selection to form the neutral data sequence (500) by means of an optimization comparison (606) based on the error between the first data and the second data in order to limit disturbance caused when the neutral data sequence (500) applied to the data frame (100), for further processing that utilizes the neutral data sequence (500) and the data frame.

2. The computer-implemented method of claim 1, **characterized by** forming (802) the neutral data sequence (500) by optimizing, in the optimization comparison (606), an error between the first and second data, the second data being formed by applying the result algorithm to the electroencephalogram data and the at least one surrogate data sequence (662), and the first data being formed by applying the result algorithm to the reference data, which corresponds to the electroencephalogram data.

3. The computer-implemeted of claim 1, **characterized by** combining (804) the data frame (100) including the electroencephalogram data and the neutral data sequence (500).

4. The computer-implemented method of claim 1, **characterized by** performing the optimization of the error by substituting a corrupted or missing data sequence (102) of the electroencephalogram data with the at least one surrogate data sequence (662) for forming the first data by applying the result algorithm to the electroencephalogram data with the at least one surrogate data sequence (662); and
replacing the corrupted or missing data sequence (102) of the electroencephalogram data with a neutral data sequence (500) formed by the optimization comparison (606).

5. The computer-implemented method of claim 1, **characterized by** receiving the electroencephalogram data from a plurality of electroencephalogram channels (N); processing the plurality of electroencephalogram channels as a vector such that a data frame (100) of a single channel of the plurality of the electroencephalogram channels is processed as a single element of the vector; and performing the optimization comparison (606) to at least one element of the vector.

6. The computer-implemented method of claim 1, **characterized by** forming a plurality of the surrogate data sequences (662) for a plurality of the data frames (100), and selecting, for each of the data frames (100), only one of the neutral data sequence (500) based on the optimization comparison.

7. The computer-implemented method of claim 1, **characterized by**
applying (1000) the result algorithm to the electroencephalogram data with the neutral data sequence for providing at least one measured parameter, the neutral data sequence (500) for the electroencephalogram data; and
outputting (1002) the at least one measured parameter.

8. The computer-implemented method of claim 1, **characterized by** forming the neutral data sequence (500) for the data frame (100) including the electroencephalogram data in real time while a measurement of the electroencephalogram data or transfer thereof is on-going.

9. The computer-implemented method of claim 1, **characterized by**
performing the selection of one surrogate algorithm (652) from at least one surrogate algorithm (652), each of which is for generating at least one surrogate data sequence (662), which includes a neutral data sequence (500), the selection being based on an optimization comparison (606) between a first data and a second data in order to limit disturbance caused by the neutral data sequence (500) applied to the data frame (100).

10. The computer-implemented method of claim 9, **characterized by** forming the surrogate data sequence (662) using the surrogate algorithm (652), which utilizes the electroencephalogram data that is uncorrupted, the surrogate algorithm (652) being dependent on the result algorithm.

11. The computer-implemented method of claim 9, **characterized by**
forming (900), based on the result algorithm, at least one first parameter, which defines the surrogate algorithm (652);
forming (902) the at least one surrogate data sequence (662) using the surrogate algorithm (652) determined by the at least one first parameter for the optimization comparison (606), the surrogate algorithm (652) being random, pseudorandom or deterministic; and
combining (904) the data frame (100) and a neutral data sequence (500) formed by the optimization (662).

12. The computer-implemented method of claim 10 or 11, **characterized by** determining the at least one first parameter of the surrogate algorithm (652) based on at least one frame parameter of the data frame (100), the at least one frame parameter defining a property of the data frame (100).

13. The computer-implemented method of claim 10, 11 or 12, **characterized by** determining the at least one first parameter of the surrogate algorithm (652) based on at least one second parameter of the result algorithm, the at least one second parameter defining the result algorithm.

14. The computer-implemented method of claim 9, **characterized by** forming, for the optimization comparison, at least one reference result by applying the result algorithm to at least one model electroencephalogram data, a single reference result corresponding to a single model electroencephalogram data of the at least one model electroencephalogram data, and/or
at least one reference algorithm that provides at least one reference result similar to those formed by applying the result algorithm to at least one model electroencephalogram data.

15. An electroencephalogram apparatus for forming modifying data for a data sequence of a data frame (100) including electroencephalogram data, **characterized in that** the electroencephalogram apparatus comprises
one or more processors (702); and
one or more memories (704) including computer program code;
the one or more memories (704) and the computer program code configured to, with the one or more processors (702), cause the electroencephalogram apparatus at least to:
form the modifying data by performing a selection of one sequence from at least one surrogate data sequence (662), where the selected one is configured to become a neutral data sequence (500), the neutral data sequence (500) replacing a missing or corrupted data sequence of the electroencephalogram data, and
form the neutral data sequence (500) by optimization, within the optimization comparison (606), of an error between a first data and a second data, wherein the first data comprises a reference result is formed by application of a result algorithm to reference data corresponding to electroencephalogram data that is non-corrupted and without missing portions, and the second data comprises at least one result formed by application of the result algorithm to the electroencephalogram data with the one sequence from the at least one surrogate data sequence (662) in the location of the corrupted or missing data sequence (102), the result algorithm providing characterizing information on the data frame (100) including the electroencephalogram data, wherein the formation of the neutral data sequence (500) comprises
perform the selection to form the neutral data sequence (500) based on an optimization comparison (606) between a first data and a second data in order to limit disturbance caused in case the neutral data sequence (500) is applied to the data frame (100).

16. The apparatus of claim 15, **characterized in that**
the one or more memories (704) and the computer program code configured to, with the one or more processors (702), cause the electroencephalogram apparatus to:
perform a selection of one surrogate algorithm (652) from at least one surrogate algorithm (652), each of which is for generating at least one surrogate data sequence (662), which includes the neutral data sequence (500), based on an optimization comparison (606) between the first data and the second data in order to limit disturbance caused when the neutral data sequence (500) is applied to the data frame (100).

## Patentansprüche

1. Computerimplementiertes Verfahren zum Bilden von modifizierenden Daten in Bezug auf eine Datensequenz für einen Datenrahmen (100), der Elektroenzephalogrammdaten einschließt, **gekennzeichnet durch**
Bilden (800) der modifizierenden Daten durch Durchführen einer Auswahl einer Sequenz aus mindestens einer Ersatzdatensequenz (662), wobei die ausgewählte zu einer neutralen Datensequenz (500) wird, wobei die neutrale Datensequenz (500) eine fehlende oder beschädigte Datensequenz der Elektroenzephalogrammdaten ersetzt, durch
Bilden (802) der neutralen Datensequenz (500) durch Optimieren eines Fehlers zwischen ersten Daten und zweiten Daten, wobei die ersten Daten ein Referenzergebnis umfassen, das durch Anwenden eines Ergebnisalgorithmus auf Referenzdaten gebildet wird, die Elektroenzephalogrammdaten entsprechen, die nicht beschädigt sind und keine fehlenden Abschnitte aufweisen, und wobei die zweiten Daten mindestens ein Ergebnis umfassen, das jeweils durch Anwenden des Ergebnisalgorithmus auf die Elektroenzephalogrammdaten mit der einen Sequenz aus der mindestens einen Ersatzdatensequenz (662) an der Stelle der beschädigten oder fehlenden Datensequenz (102) gebildet wird, wobei der Ergebnisalgorithmus charakterisierende Informationen über den Datenrahmen (100) bereitstellt, der die Elektroenzephalogrammdaten einschließt, wobei das Bilden der neutralen Datensequenz (500) umfasst:
Durchführen der Auswahl zum Bilden der neutralen Datensequenz (500) mittels eines Optimierungsvergleichs (606) auf der Grundlage des Fehlers zwischen den ersten Daten und den zweiten Daten, um eine Störung zu begrenzen, die verursacht wird, wenn die neutrale Datensequenz (500) auf den Datenrahmen (100) angewendet wird, zur weiteren Verarbeitung, die die neutrale Datensequenz (500) und den Datenrahmen nutzt.

2. Computerimplementiertes Verfahren nach Anspruch 1, **gekennzeichnet durch** Bilden (802) der neutralen Datensequenz (500) durch Optimieren eines Fehlers zwischen den ersten und zweiten Daten in dem Optimierungsvergleich (606),
wobei die zweiten Daten durch Anwenden des Ergebnisalgorithmus auf die Elektroenzephalogrammdaten und die mindestens eine Ersatzdatensequenz (662) gebildet werden, und
wobei die ersten Daten durch Anwenden des Ergebnisalgorithmus auf die Referenzdaten gebildet werden, die den Elektroenzephalogrammdaten entsprechen.

3. Computerimplementiertes nach Anspruch 1, **gekennzeichnet durch** Kombinieren (804) des Datenrahmens (100), der die Elektroenzephalogrammdaten einschließt, und der neutralen Datensequenz (500).

4. Computerimplementiertes Verfahren nach Anspruch 1, **gekennzeichnet durch** Durchführen der Optimierung des Fehlers durch Ersetzen einer beschädigten oder fehlenden Datensequenz (102) der Elektroenzephalogrammdaten durch die mindestens eine Ersatzdatensequenz (662) zum Bilden der ersten Daten durch Anwenden des Ergebnisalgorithmus auf die Elektroenzephalogrammdaten mit der mindestens einen Ersatzdatensequenz (662); und
Ersetzen der beschädigten oder fehlenden Datensequenz (102) der Elektroenzephalogrammdaten durch eine neutrale Datensequenz (500), die durch den Optimierungsvergleich (606) gebildet wird.

5. Computerimplementiertes Verfahren nach Anspruch 1, **gekennzeichnet durch** Empfangen der Elektroenzephalogrammdaten von einer Mehrzahl von Elektroenzephalogrammkanälen (N);
Verarbeiten der Mehrzahl von Elektroenzephalogrammkanälen als einen Vektor, sodass ein Datenrahmen (100) eines einzelnen Kanals der Mehrzahl von Elektroenzephalogrammkanälen als ein einzelnes Element des Vektors verarbeitet wird; und
Durchführen des Optimierungsvergleichs (606) für mindestens ein Element des Vektors.

6. Computerimplementiertes Verfahren nach Anspruch 1, **gekennzeichnet durch** Bilden einer Mehrzahl der Ersatzdatensequenzen (662) für eine Mehrzahl der Datenrahmen (100) und Auswählen, für jeden der Datenrahmen (100), nur einer der neutralen Datensequenzen (500) auf der Grundlage des Optimierungsvergleichs.

7. Computerimplementiertes Verfahren nach Anspruch 1, **gekennzeichnet durch**
Anwenden (1000) des Ergebnisalgorithmus auf die Elektroenzephalogrammdaten mit der neutralen Datensequenz zum Bereitstellen mindestens eines gemessenen Parameters, die neutrale Datensequenz (500) für die Elektroenzephalogrammdaten; und
Ausgeben (1002) des mindestens einen gemessenen Parameters.

8. Computerimplementiertes Verfahren nach Anspruch 1, **gekennzeichnet durch** Bilden der neutralen Datensequenz (500) für den Datenrahmen (100), der die Elektroenzephalogrammdaten einschließt, in Echtzeit, während eine Messung der Elektroenzephalogrammdaten oder eine Übertragung davon stattfindet.

9. Computerimplementiertes Verfahren nach Anspruch 1, **gekennzeichnet durch**
Durchführen der Auswahl eines Ersatzalgorithmus (652) aus mindestens einem Ersatzalgorithmus (652), von denen jeder zum Erzeugen mindestens einer Ersatzdatensequenz (662) vorgesehen ist, die eine neutrale Datensequenz (500) einschließt,
wobei die Auswahl auf einem Optimierungsvergleich (606) zwischen ersten Daten und zweiten Daten basiert, um eine Störung zu begrenzen, die durch die auf den Datenrahmen (100) angewendete neutrale Datensequenz (500) verursacht wird.

10. Computerimplementiertes Verfahren nach Anspruch 9, **gekennzeichnet durch** Bilden der Ersatzdatensequenz (662) unter Verwendung des Ersatzalgorithmus (652), der die unbeschädigten Elektroenzephalogrammdaten nutzt, wobei der Ersatzalgorithmus (652) von dem Ergebnisalgorithmus abhängig ist.

11. Computerimplementiertes Verfahren nach Anspruch 9, **gekennzeichnet durch**
Bilden (900), auf der Grundlage des Ergebnisalgorithmus, mindestens eines ersten Parameters, der den Ersatzalgorithmus (652) definiert;
Bilden (902) der mindestens einen Ersatzdatensequenz (662) unter Verwendung des Ersatzalgorithmus (652), der durch den mindestens einen ersten Parameter für den Optimierungsvergleich (606) bestimmt wird, wobei der Ersatzalgorithmus (652) zufällig, pseudozufällig oder deterministisch ist; und
Kombinieren (904) des Datenrahmens (100) und einer neutralen Datensequenz (500), die durch die Optimierung (662) gebildet wird.

12. Computerimplementiertes Verfahren nach Anspruch 10 oder 11, **gekennzeichnet durch** Bestimmen des mindestens einen ersten Parameters des Ersatzalgorithmus (652) auf der Grundlage mindestens eines Rahmenparameters des Datenrahmens (100), wobei der mindestens eine Rahmenparameter eine Eigenschaft des Datenrahmens (100) definiert.

13. Computerimplementiertes Verfahren nach Anspruch 10, 11 oder 12, **gekennzeichnet durch** Bestimmen des mindestens einen ersten Parameters des Ersatzalgorithmus (652) auf der Grundlage mindestens eines zweiten Parameters des Ergebnisalgorithmus, wobei der mindestens eine zweite Parameter den Ergebnisalgorithmus definiert.

14. Computerimplementiertes Verfahren nach Anspruch 9, **gekennzeichnet durch** Bilden mindestens eines Referenzergebnisses für den Optimierungsvergleich durch Anwenden des Ergebnisalgorithmus auf mindestens eine Modell-Elektroenzephalogrammdate, wobei ein einzelnes Referenzergebnis einer einzelnen Modell-Elektroenzephalogrammdate der mindestens einen Modell-Elektroenzephalogrammdate entspricht, und/oder
mindestens eines Referenzalgorithmus, der mindestens ein Referenzergebnis bereitstellt, das jenen ähnelt, die durch Anwenden des Ergebnisalgorithmus auf mindestens eine Modell-Elektroenzephalogrammdate gebildet werden.

15. Elektroenzephalogrammeinrichtung zum Bilden von modifizierenden Daten für eine Datensequenz eines Datenrahmens (100), der Elektroenzephalogrammdaten einschließt, **dadurch gekennzeichnet, dass** die Elektroenzephalogrammeinrichtung umfasst:
einen oder mehrere Prozessoren (702); und
einen oder mehrere Speicher (704), die Computerprogrammcode einschließen;
wobei der eine oder die mehreren Speicher (704) und der Computerprogrammcode dazu ausgebildet sind, mit dem einen oder den mehreren Prozessoren (702) die Elektroenzephalogrammeinrichtung zumindest zu veranlassen zum:
Bilden der modifizierenden Daten durch Durchführen einer Auswahl einer Sequenz aus mindestens einer Ersatzdatensequenz (662), wobei die ausgewählte dazu ausgebildet ist, zu einer neutralen Datensequenz (500) zu werden, wobei die neutrale Datensequenz (500) eine fehlende oder beschädigte Datensequenz der Elektroenzephalogrammdaten ersetzt, und
Bilden der neutralen Datensequenz (500) durch Optimierung, in dem Optimierungsvergleich (606), eines Fehlers zwischen ersten Daten und zweiten Daten, wobei die ersten Daten ein Referenzergebnis umfassen, das durch Anwendung eines Ergebnisalgorithmus auf Referenzdaten gebildet wird, die Elektroenzephalogrammdaten entsprechen, die nicht beschädigt sind und keine fehlenden Abschnitte aufweisen,
und wobei die zweiten Daten mindestens ein Ergebnis umfassen, das durch Anwendung des Ergebnisalgorithmus auf die Elektroenzephalogrammdaten mit der einen Sequenz aus der mindestens einen Ersatzdatensequenz (662) an der Stelle der beschädigten oder fehlenden Datensequenz (102) gebildet wird,
wobei der Ergebnisalgorithmus charakterisierende Informationen über den Datenrahmen (100) bereitstellt, der die Elektroenzephalogrammdaten einschließt, wobei die Bildung der neutralen Datensequenz (500) umfasst:
Durchführen der Auswahl zum Bilden der neutralen Datensequenz (500) auf der Grundlage eines Optimierungsvergleichs (606) zwischen ersten Daten und zweiten Daten, um eine Störung zu begrenzen, die verursacht wird, wenn die neutrale Datensequenz (500) auf den Datenrahmen (100) angewendet wird.

16. Einrichtung nach Anspruch 15, **dadurch gekennzeichnet, dass**
der eine oder die mehreren Speicher (704) und der Computerprogrammcode dazu ausgebildet sind, mit dem einen oder den mehreren Prozessoren (702) die Elektroenzephalogrammeinrichtung zu veranlassen zum:
Durchführen einer Auswahl eines Ersatzalgorithmus (652) aus mindestens einem Ersatzalgorithmus (652), von denen jeder zum Erzeugen mindestens einer Ersatzdatensequenz (662) vorgesehen ist, die die neutrale Datensequenz (500) einschließt, auf der Grundlage eines Optimierungsvergleichs (606) zwischen den ersten Daten und den zweiten Daten, um eine Störung zu begrenzen, die verursacht wird, wenn die neutrale Datensequenz (500) auf den Datenrahmen (100) angewendet wird.

## Revendications

1. Procédé mis en œuvre par ordinateur de formation de données de modification relatives à une séquence de données pour une trame (100) de données incluant des données d'électroencéphalogramme, **caractérisé par**
la formation (800) des données de modification par réalisation d'une sélection d'une séquence parmi au moins une séquence de données de substitution (662), où l'une sélectionnée devient une séquence de données neutres (500), la séquence de données neutres (500) remplaçant une séquence de données manquantes ou corrompues des données d'électroencéphalogramme, par
la formation (802) de la séquence de données neutres (500) par optimisation d'une erreur entre des premières données et des deuxièmes données, dans lequel les premières données comprennent un résultat de référence formé par application d'un algorithme de résultat à des données de référence, lesquelles correspondent à des données d'électroencéphalogramme qui sont non corrompues et sans parties manquantes, et les deuxièmes données comprennent au moins un résultat chacun formé par application de l'algorithme de résultat aux données d'électroencéphalogramme avec l'une séquence parmi l'au moins une séquence de données de substitution (662) à l'emplacement de la séquence de données corrompues ou manquantes (102), l'algorithme de résultat fournissant des informations de caractérisation sur la trame (100) de données incluant les données d'électroencéphalogramme, dans lequel la formation de la séquence de données neutres (500) comprend
la réalisation de la sélection pour former la séquence de données neutres (500) au moyen d'une comparaison d'optimisation (606) sur la base de l'erreur entre les premières données et les deuxièmes données afin de limiter une perturbation causée lorsque la séquence de données neutres (500) est appliquée à la trame (100) de données, pour un traitement ultérieur qui utilise la séquence de données neutres (500) et la trame de données.

2. Procédé mis en œuvre par ordinateur selon la revendication 1, **caractérisé par** la formation (802) de la séquence de données neutres (500) par optimisation, dans la comparaison d'optimisation (606), d'une erreur entre les premières et deuxièmes données, les deuxièmes données étant formées par application de l'algorithme de résultat aux données d'électroencéphalogramme et à l'au moins une séquence de données de substitution (662), et les premières données étant formées par application de l'algorithme de résultat aux données de référence, lesquelles correspondent aux données d'électroencéphalogramme.

3. Procédé mis en œuvre par ordinateur selon la revendication 1, **caractérisé par** la combinaison (804) de la trame (100) de données incluant les données d'électroencéphalogramme et de la séquence de données neutres (500).

4. Procédé mis en œuvre par ordinateur selon la revendication 1, **caractérisé par** la réalisation de l'optimisation de l'erreur en substituant une séquence de données corrompues ou manquantes (102) des données d'électroencéphalogramme par l'au moins une séquence de données de substitution (662) pour former les premières données par application de l'algorithme de résultat aux données d'électroencéphalogramme avec l'au moins une séquence de données de substitution (662) ; et,
le remplacement de la séquence de données corrompues ou manquantes (102) des données d'électroencéphalogramme par une séquence de données neutres (500) formée par la comparaison d'optimisation (606).

5. Procédé mis en œuvre par ordinateur selon la revendication 1, **caractérisé par** la réception des données d'électroencéphalogramme d'une pluralité de canaux (N) d'électroencéphalogramme ;
le traitement de la pluralité de canaux d'électroencéphalogramme comme un vecteur de sorte qu'une trame (100) de données d'un canal unique de la pluralité des canaux d'électroencéphalogramme est traitée comme un élément unique du vecteur ; et
la réalisation de la comparaison d'optimisation (606) sur au moins un élément du vecteur.

6. Procédé mis en œuvre par ordinateur selon la revendication 1, **caractérisé par** la formation d'une pluralité de séquences de données de substitution (662) pour une pluralité de trames (100) de données, et la sélection, pour chacune des trames (100) de données, d'une seule de la séquence de données neutres (500) sur la base de la comparaison d'optimisation.

7. Procédé mis en œuvre par ordinateur selon la revendication 1, **caractérisé par**
l'application (1000) de l'algorithme de résultat aux données d'électroencéphalogramme avec la séquence de données neutres pour fournir au moins un paramètre mesuré, la séquence de données neutres (500) pour les données d'électroencéphalogramme ; et
la sortie (1002) de l'au moins un paramètre mesuré.

8. Procédé mis en œuvre par ordinateur selon la revendication 1, **caractérisé par** la formation de la séquence de données neutres (500) pour la trame (100) de données incluant les données d'électroencéphalogramme en temps réel tandis qu'une mesure des données d'électroencéphalogramme ou le transfert de celles-ci est en cours.

9. Procédé mis en œuvre par ordinateur selon la revendication 1, **caractérisé par**
la réalisation de la sélection d'un algorithme de substitution (652) parmi au moins un algorithme de substitution (652), dont chacun est destiné à générer au moins une séquence de données de substitution (662), qui inclut une séquence de données neutres (500), la sélection étant sur basée sur une comparaison d'optimisation (606) entre des premières données et des deuxièmes données afin de limiter une perturbation causée par la séquence de données neutres (500) appliquée à la trame (100) de données.

10. Procédé mis en œuvre par ordinateur selon la revendication 9, **caractérisé par** la formation de la séquence de données de substitution (662) à l'aide de l'algorithme de substitution (652), qui utilise les données d'électroencéphalogramme qui sont non corrompues, l'algorithme de substitution (652) étant dépendant de l'algorithme de résultat.

11. Procédé mis en œuvre par ordinateur selon la revendication 9, **caractérisé par**
la formation (900), sur la base de l'algorithme de résultat, d'au moins un premier paramètre, qui définit l'algorithme de substitution (652) ;
la formation (902) de l'au moins une séquence de données de substitution (662) à l'aide de l'algorithme de substitution (652) déterminé par l'au moins un premier paramètre pour la comparaison d'optimisation (606), l'algorithme de substitution (652) étant aléatoire, pseudo-aléatoire ou déterministe ; et
la combinaison (904) de la trame (100) de données et d'une séquence de données neutres (500) formée par l'optimisation (662).

12. Procédé mis en œuvre par ordinateur selon la revendication 10 ou la revendication 11, **caractérisé par** la détermination de l'au moins un premier paramètre de l'algorithme de substitution (652) sur la base d'au moins un paramètre de trame de la trame (100) de données, l'au moins un paramètre de trame définissant une propriété de la trame (100) de données.

13. Procédé mis en œuvre par ordinateur selon la revendication 10, la revendication 11 ou la revendication 12, **caractérisé par** la détermination de l'au moins un premier paramètre de l'algorithme de substitution (652) sur la base d'au moins un deuxième paramètre de l'algorithme de résultat, l'au moins un deuxième paramètre définissant l'algorithme de résultat.

14. Procédé mis en œuvre par ordinateur selon la revendication 9, **caractérisé par** la formation, pour la comparaison d'optimisation, d'au moins un résultat de référence par application de l'algorithme de résultat à au moins une donnée d'électroencéphalogramme de modèle, un résultat de référence unique correspondant à une seule donnée d'électroencéphalogramme de modèle de l'au moins une donnée d'électroencéphalogramme de modèle, et/ou
au moins un algorithme de référence qui fournit au moins un résultat de référence similaire à ceux formés par application de l'algorithme de résultat à au moins une donnée d'électroencéphalogramme de modèle.

15. Appareil d'électroencéphalogramme pour la formation de données de modification pour une séquence de données d'une trame (100) de données incluant des données d'électroencéphalogramme, **caractérisé en ce que** l'appareil d'électroencéphalogramme comprend
un ou plusieurs processeurs (702) ; et
une ou plusieurs mémoires (704) incluant un code de programme informatique ;
les une ou plusieurs mémoires (704) et le code de programme informatique étant configurés pour, avec les un ou plusieurs processeurs (702), amener l'appareil d'électroencéphalogramme au moins à :
former les données de modification par réalisation d'une sélection d'une séquence parmi au moins une séquence de données de substitution (662), où l'une sélectionnée est configurée pour devenir une séquence de données neutres (500), la séquence de données neutres (500) remplaçant une séquence de données corrompues ou manquantes des données d'électroencéphalogramme, et
former la séquence de données neutres (500) par optimisation, au sein de la comparaison d'optimisation (606), d'une erreur entre des premières données et des deuxièmes données, dans lequel les premières données comprennent un résultat de référence est formé par application d'un algorithme de résultat à des données de référence correspondant à des données d'électroencéphalogramme qui sont non corrompues et sans parties manquantes, et les deuxièmes données comprennent au moins un résultat formé par application de l'algorithme de résultat aux données d'électroencéphalogramme avec l'une séquence parmi l'au moins une séquence de données de substitution (662) à l'emplacement de la séquence de données corrompues ou manquantes (102), l'algorithme de résultat fournissant des informations de caractérisation sur la trame (100) de données incluant les données d'électroencéphalogramme, dans lequel la formation de la séquence de données neutres (500) comprend
la réalisation de la sélection pour former la séquence de données neutres (500) sur la base d'une comparaison d'optimisation (606) entre des premières données et des deuxièmes données afin de limiter une perturbation causée dans le cas où la séquence de données neutres (500) est appliquée à la trame (100) de données.

16. Appareil selon la revendication 15, **caractérisé en ce que**
les une ou plusieurs mémoires (704) et le code de programme informatique sont configurés pour, avec les un ou plusieurs processeurs (702), amener l'appareil d'électroencéphalogramme à :
réaliser une sélection d'un algorithme de substitution (652) parmi au moins un algorithme de substitution (652), dont chacun est destiné à générer au moins une séquence de données de substitution (662), qui inclut la séquence de données neutres (500), sur la base d'une comparaison d'optimisation (606) entre les premières données et les deuxièmes données afin de limiter une perturbation causée lorsque la séquence de données neutres (500) est appliquée à la trame (100) de données.
